Europäisches Patentamt

European Patent Office

Office européen des brevets

(19)

(11) Veröffentlichungsnummer: **0 206 194**
**B1**

(12) **EUROPÄISCHE PATENTSCHRIFT**

(45) Veröffentlichungstag der Patentschrift:
02.05.90

(51) Int. Cl.⁴: **C07D 307/60**

(21) Anmeldenummer: **86108180.0**

(22) Anmeldetag: **14.06.86**

(54) **Verfahren zur kontinuierlichen Abscheidung von Maleinsäureanhydrid aus gasförmigen Reaktionsgemischen.**

(30) Priorität: **19.06.85 DE 3521769**

(43) Veröffentlichungstag der Anmeldung:
**30.12.86 Patentblatt 86/52**

(45) Bekanntmachung des Hinweises auf die Patenterteilung:
**02.05.90 Patentblatt 90/18**

(84) Benannte Vertragsstaaten:
**BE DE FR GB IT NL**

(56) Entgegenhaltungen:
**EP-A- 0 149 144**

(73) Patentinhaber: **BASF Aktiengesellschaft,
Carl-Bosch-Strasse 38, D-6700 Ludwigshafen(DE)**

(72) Erfinder: **Malsch, Klaus-Dieter, Dr., Amselweg 14,
D-6707 Schifferstadt(DE)**
Erfinder: **Fischer, Rolf, Dr., Bergstrasse 98,
D-6900 Heidelberg(DE)**
Erfinder: **Schnabel, Rolf, Dr., Frankenstrasse 22,
D-6707 Schifferstadt(DE)**

## Beschreibung

Diese Erfindung betrifft ein Verfahren zur kontinuierlichen Abscheidung von Maleinsäureanhydrid (MSA) aus gasförmigen Reaktionsgemischen.

Aus der DE-OS 2 543 673 ist bekannt, daß man 1,4-Butandiol aus MSA dadurch herstellen kann, daß man in einer ersten Stufe MSA mit einem Butanol verestert und den Maleinsäuredibutylester in einer zweiten Stufe katalytisch hydriert. Nach einem in der DE-PS 2 845 905 beschriebenen Verfahren führt man die 1,4-Butandiol-Bildung in einer Stufe durch, wobei man ein Gemisch aus MSA und dem Alkohol unter Verzicht auf eine vollständige Veresterung des MSA unmittelbar katalytisch hydriert.

In der DE-OS 3 106 819 wird der Vorschlag gemacht, das MSA aus einem gasförmigen Reaktionsgemisch durch eine Wäsche mit 1,4-Butandiol abzuscheiden. Das dabei erhältliche Gemisch wird zum 1,4-Butandiol hydriert. Man muß hierbei zwar kein Lösungsmittel abtrennen, doch gelingt es nicht, sowohl hohe Konzentrationen an MSA bzw. an monomeren MSA-Folgeprodukten im Absorbat zu erhalten als auch die unerwünschte Bildung von Oligomeren und Polymeren aus Butandiol und MSA zu verhindern. Beides ist aber wünschenswert, denn hohe Raum-Zeit-Ausbeuten kann man nur mit hohen Absorbat-Konzentrationen erzielen, und Oligomere und Polymere beeinträchtigen die Katalysatoren bei der nachfolgenden Hydrierung.

Versucht man, für eine Butandiol-Herstellung geeignete Gemische aus Butanol und MSA herzustellen, so ergeben sich durch den hohen Gehalt des Abgases an Butanol so erhebliche technische Schwierigkeiten, daß eine Butanolwäsche mit wirtschaftlich vertretbarem Aufwand nicht betrieben werden kann. Ein Verfahren zur kontinuierlichen Abscheidung von MSA aus Reaktionsgasen mit Butanol, bei dem man die dabei anfallenden gasförmigen Stoffe im Gegenstrom mit Maleinsäuredibutylester in Berührung bringt, und wobei man ein flüssiges Gemisch erhält, das sich vorteilhaft zur Gewinnung von 1,4-Butandiol hydrieren läßt, wird in der älteren, nicht vorveröffentlichten EP-A 149 144 beschrieben.

Bei dem Verfahren der Erfindung, das die Gewinnung von MSA auf besonders wirtschaftliche Weise erlaubt, nimmt man die kontinuierliche Abscheidung von MSA aus gasförmigen Reaktionsgemischen, die man bei der katalytischen Oxidation von Kohlenwasserstoffen erhält, durch Behandlung des gasförmigen Reaktionsgemischs mit Lösungsmitteln so vor, daß man das MSA enthaltende gasförmige Reaktionsgemisch mit Butanol in Kontakt bringt, die dabei anfallenden gasförmigen Stoffe im Gegenstrom mit Fumarsäuredibutylester oder Bernsteinsäuredibutylester in Berührung bringt und das flüssige Verfahrensprodukt dem Sumpf entnimmt.

Nach dem erfindungsgemäßen Verfahren bringt man ein MSA enthaltendes gasförmiges Reaktionsgemisch zum Zwecke der MSA-Absorption mit dem Butanol in Kontakt. Die Reaktionsgaszufuhr erfolgt bei einer zweckmäßigen Ausführungsform des Verfahrens unterhalb der Oberfläche des flüssigen Butanols, z.B. durch ein Tauchrohr. Das Reaktionsgemisch kann aber auch direkt von unten in eine Absorptionskolonne eingeleitet werden, wo ihm flüssiges Butanol entgegenströmt. Die Arbeitsweise, bei der man die MSA-Absorption in einer Kolonne oder in mehreren hintereinandergeschalteten Kolonnen durchführt, ist bevorzugt. Man kann das Reaktionsgemisch auch mit gasförmigem Butanol in Kontakt bringen.

MSA enthaltende Reaktionsgemische werden z.B. nach der an sich bekannten katalytischen Oxidation von Kohlenwasserstoffen wie Butenen, Butan oder auch Benzol, erhalten. Bei diesem bekannten Oxidationsverfahren strömt aus dem Reaktor ein gasförmiges Reaktionsgemisch mit einer Temperatur von 250 bis 600°C, das je Nm³ bis 40 g Maleinsäureanhydrid enthält. Außerdem enthalten die gasförmigen Reaktionsgemische neben nicht umgesetztem Kohlenwasserstoff Wasser, Kohlenmonoxid und Kohlendioxid.

Als Kolonnen sind z.B. Absorptionskolonnen, wie Glockenboden-Füllkörper- und Siebbodenkolonnen mit Bodenzahlen von 1 bis 50, vorzugsweise 5 bis 20 geeignet. Man arbeitet zweckmäßigerweise so, daß man das das MSA enthaltende gasförmige Reaktionsgemisch in den Kolonnensumpf, Butanol in den unteren Teil der Kolonne, vorteilhaft oberhalb des Kolonnensumpfes und den Dibutylester über den Kopf in die Kolonne einleitet. Die Temperaturen liegen im Sumpf bei 0 bis 280°C, in der Kolonne bei -20 bis 150°C und am Kopf der Kolonne bei -20 bis 130°C. Als Butanole kommen n- oder iso-Butanol in Frage; als Dibutylester verwendet man den Bernstein- oder Fumarsäuredibutylester des eingesetzten Alkohols.

Bezogen auf 1 Mol MSA im gasförmigen Ausgangsgemisch wendet man 0,2 bis 10 Mol Butanol und 0,2 bis 20 Mol des Dibutylesters an.

Nach einer bevorzugten Arbeitsweise, bei der man die kontinuierliche Abscheidung des MSA in einer Kolonne vornimmt, werden das Gasgemisch und das Butanol wie geschildert in den Sumpf bzw. den unteren Teil der Kolonne eingeleitet. Als unterer Teil der Kolonne wird der Teil oberhalb des Sumpfes bis zum zweiten bis fünften Boden verstanden.

Oberhalb der Einleitung des Butanols, also oberhalb des zweiten bis fünften Bodens, wird eine eventuell auftretende zweite flüssige Phase, die hauptsächlich aus Wasser besteht, aus der Kolonne ausgeschleust. Aus dem mittleren Teil der Kolonne, unter dem der Bereich bis zu fünf Böden oberhalb der Entnahmestellen für die wäßrige Phase verstanden wird, schleust man ein flüssiges Gemisch aus, aus dem man in einer separaten Destillationsvorrichtung, wie einer Destillationskolonne, die leichter als der Dibutylester siedenden Anteile abdestilliert, die man in den unteren Teil der Kolonne zurückführt. Der bei dieser separaten Destillation als Sumpfprodukt anfallende Dibutylester wird der Absorptionskolonne über Kopf wieder zugeführt. Die Kolonne wird dabei so betrieben, daß die Temperatur im untersten Boden zwischen 0 und 120°C, vorzugsweise zwischen 25 und 100°C liegt, sich im mittleren Teil der Kolonne eine Temperatur von -20 bis 100°C, vorzugsweise von 0 bis 65°C und am Ko-

Ionnenkopf eine Temperatur von -20 bis 50°C, vorzugsweise -20 bis 30°C einstellt.

Unter den Bedingungen der erfindungsgemäßen Absorption wird das MSA überwiegend in die entsprechenden Mono- und -dibutylester überführt. Die gasförmigen Begleitstoffe des MSA werden über den Kopf der Kolonne abgeleitet, während eventuell ein Teil des Wassers die Kolonne durch den Seitenabzug verläßt. Das flüssige Verfahrensprodukt, das man dem Sumpf der Kolonne entnimmt, hat z.B. folgende Zusammensetzung: 0,01 bis 93 Gew.% Butanol, 0,7 bis 79 Gew.% Maleinsäuremonobutylester, 1 bis 95 Gew.% Maleinsäuredibutylester, bis zu 50 Gew.% MSA, Maleinsäure und Fumarsäure. Der Maleinsäuredibutylester enthält gewöhnlich erhebliche Anteile an dem isomeren Fumarsäuredibutylester. Das flüssige Gemisch eiget sich hervorragend für die Hydrierung zur Gewinnung von 1,4-Butandiol. Hierzu empfiehlt es sich, das flüssige Gemisch zum Zwecke der Vervollständigung der Veresterung z.B. auf 110 bis 250°C zu erhitzen, wobei man das Reaktionswasser mit dem überschüssigen Butanol abtreibt und somit das für das erfindungsgemäße Verfahren benötigten Butanol zurückgewinnt. Der Maleinsäuredibutylester kann dann auf an sich bekannte Weise zu 1,4-Butandiol hydriert werden.

Das neue Verfahren ermöglicht es, das MSA aus den Reaktionsgasen quantitativ und unter weitgehender Vermeidung von Absorptionsmittel-Verlusten in ein flüssiges Gemisch zu überführen, das eine hohe Konzentration an dem Halb- und Diester der Maleinsäure aufweist und das sich hervorragend für die Hydrierung zur Gewinnung von 1,4-Butandiol eignet. Überraschenderweise treten bei der erfindungsgemäßen Arbeitsweise störende Abscheidungen von Kristallen aus Maleinsäure und/oder Fumarsäure nicht auf.

Beispiel 1

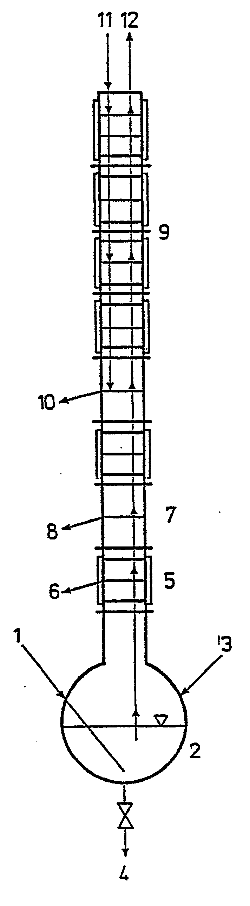

(siehe Figur 1)
Ein Stickstoffstrom von 200 Nl/h (Normalliter pro Stunde) wird in einem Sättigungsgefäß mit MSA und mit Waserdampf so beladen, daß er 0,5 Vol.% MSA (4,5 g/h) und 5 Vol.% Waser enthält. Der so hergestellte Gasstrom entspricht bezüglich dieser Komponenten weitgehend einem gasförmigen Reaktionsgemisch, das man bei der bekannten Luftoxidation von Butan, Butenen oder auch Benzol zum Zwecke der Herstellung von MSA erhält.

Das Gasgemisch (1) wird in einem zu Versuchsbeginn mit 1/2 Liter Butanol beschickten Kolben (2) bei einer Temperatur von 60°C getaucht eingeleitet. Der Kolben ist mit einer Zuleitung (3) ausgerüstet. Durch eine Ableitung (4) wird in regelmäßigen Zeitabständen flüssiges Reaktionsgemisch entnommen, so daß der Flüssigkeitsstand im Kolben konstant bleibt. Auf dem Kolben ist ein Schuß einer Glockenbodenkolonne (5) mit drei Glockenböden angebracht, der mit einem Probeentnahmestutzen (6) ausgerüstet ist. Die Temperatur hält man in diesem Teil der Kolonne auf 50°C. Über die Zuleitung (3) werden 10 ml/h n-Butanol eingeleitet. Auf dem Kolonnenteil (5) befindet sich ein mit einem Ablaßstutzen (8) versehener auf 25°C gehaltener Phasenabscheider (7), durch den man eine möglicherweise auftretende zweite flüssige Phase mit höherer Dichte (wäßrige Phase) aus der Kolonne ausschleusen kann. Auf dem Phasenabscheider (7) sind weitere Schüsse mit insgesamt 9 Glockenböden (9) angebracht, die auf 10°C gehalten werden.

Drei Böden über dem Phasenabscheider (7) ist ein Ablaßstutzen (10) installiert, mit dem man das flüssige Absorbat aus dem darüberliegenden Kolonnenteil ausschleust und einer Destillationsvorrichtung zuführt. Über den Kopf der Kolonne werden 20 ml/h Bernsteinsäuredibutylester zugeführt (11). Gleichzeitig verläßt ein Gasstrom den Kolonnenkopf (12).

Nach viertägiger Versuchsdauer stellt sich ein stationärer Zustand ein, und man erhält die folgenden Ergebnisse:
Das gasförmig zugeführte MSA (4,5 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (4) wird folgende Zusammensetzung ermittelt: 39,6 Gew.% n-Butanol, 42 Gew.% Maleinsäuremonobutylester, 12,1 Gew.% Maleinsäuredibutylester und 4,1 Gew.% MSA, Maleinsäure und Fumarsäure. Bereits am Ablaßstutzen (6) lassen sich weder MSA, Maleinsäure, Fumarsäure noch Halbester nachweisen. Am Phasenabscheider (7) werden 5 bis 6 g/h einer wäßrigen Phase ausgeschleust, die 6 bis 7 Gew.% Butanol enthält. Das über den Ablaßstutzen (10) entnommene flüssige Absorbat enthält 6,8 Gew.% n-Butanol und 92 Gew.% Bernsteinsäuredibutylester sowie geringe Mengen Wasser. Die Butanol-Konzentration geht im oberen Teil der Kolonne (9) bis auf 2,2 Gew.% zurück. Mit dem Abgas (12) werden neben 1,8 g/h Wasser 0,08 g/h n-Butanol ausgetragen.

Beispiel 2

(s. Figur)
Man verfährt wie in Beispiel 1 beschrieben, wobei man jedoch anstelle von Bernsteinsäuredibutylester Fumarsäuredibutylester zur Abgaswäsche (11) verwendet. Der Teil der Kolonne oberhalb des Phasenabscheiders (7) wird auf 25°C gehalten. Nach viertägiger Versuchsdauer stellt sich ein stationärer Zustand ein, und man erhält die folgenden Ergebnisse:
Das gasförmig zugeführte MSA (4,5 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (4) wird folgende Zusammensetzung ermittelt: 20,9 Gew.% n-Butanol, 66 Gew.% Maleinsäuremonobutylester, 13,6 Gew.% Maleinsäuredibutylester und 8,0 Gew.% MSA, Maleinsäure und Fumarsäure. Bereits am Ablaßstutzen (6) lassen sich weder MSA, Maleinsäure, Fumarsäure noch Halbester nachweisen. Am Phasenabscheider (7) werden 3 bis 4 g/h einer wäßrigen Phase ausgeschleust, die 7 bis 8 Gew.% Butanol enthält. Das über den Ablaßstutzen (10) entnommene flüssige Absorbat enthält 16,3 Gew.% n-Butanol, 81 Gew.% Fumarsäuredibutylester und geringe Mengen Wasser. Die Butanol-Konzentration geht im oberen Teil der Kolonne (9) bis auf 7,7 Gew.% zurück. Mit dem Abgas (12) werden neben 5,6 g/h Wasser 1,82 g/h n-Butanol ausgetragen.

Beispiel 3

(s. Figur, Vergleichsversuch)
Man verfährt wie in Beispiel 1 beschrieben, wobei man jedoch auf die Zufuhr von Butanol (3) verzichtet und den am Kopf der Kolonne zugeführten Diester (11) nicht über den Ablaßstutzen (10) am oberen Teil der Kolonne, sondern über die Ableitung (4) am Kolben (2) wieder entnimmt.

Innerhalb eines Tages kristallisiert Maleinsäure in der Kolonne aus, so daß die Absorption abgebrochen werden muß. Erhöht man in dem Versuch die Zugabe des Diesters auf 100 ml/h, so läßt sich die störende Kristallbildung zwar verhindern, aber man erhält dabei ein flüssiges Verfahrensprodukt, das vor einer Hydrierung zum Zwecke der Herstellung von 1,4-Butandiol eine zusätzliche Anreicherungsstufe erforderlich machen würde.

**Patentansprüche**

1. Verfahren zur kontinuierlichen Abscheidung von Maleinsäureanhydrid aus gasförmigen Reaktionsgemischen, die man bei der katalytischen Oxidation von Kohlenwasserstoffen erhält, durch Behandlung der gasförmigen Reaktionsgemische mit Lösungsmitteln, dadurch gekennzeichnet, daß man das das Maleinsäureanhydrid enthaltende gasförmige Reaktionsgemisch mit Butanol in Kontakt bringt, die dabei anfallenden gasförmigen Stoffe im Gegenstrom mit Bernsteinsäuredibutylester oder Fumarsäuredibutylester in Berührung bringt und das flüssige Verfahrensprodukt dem Sumpf entnimmt.

2. Verfahren nach Anspruch 1, dadurch gekennzeichnet, daß man, bezogen auf 1 Mol Maleinsäureanhydrid, 0,2 bis 10 Mol Butanol und 0,2 bis 20 Mol Bernstein- oder Fumarsäuredibutylester anwendet.

3. Verfahren nach Anspruch 1, dadurch gekennzeichnet, daß man das das Maleinsäureanhydrid enthaltende gasförmige Reaktionsgemisch und das Butanol in den unteren Teil einer Kolonne einleitet, Wasser oberhalb dieser Einleitung aus der Kolonne ausschleust, den Bernstein- oder Fumarsäuredibutylester über den Kopf der Kolonne zuführt und das flüssige Verfahrensprodukt dem Kolonnensumpf entnimmt.

4. Verfahren nach Anspruch 1, dadurch gekennzeichnet, daß man das das Maleinsäureanhydrid enthaltende gasförmige Reaktionsgemisch und das Butanol in den unteren Teil einer Kolonne einleitet, aus dem mittleren Teil der Kolonne flüssiges Gemisch ausschleust, aus diesem Gemisch leichter als Bernstein- oder Fumarsäuredibutylester siedende Anteile abdestilliert und in den unteren Teil der Kolonne einleitet, und den bei dieser Destillation als Sumpfprodukt anfallenden Dibutylester über den Kopf der Kolonne zuführt.

**Claims**

1. A process for continuously separating maleic anhydride from a gaseous reaction mixture obtained in the catalytic oxidation of a hydrocarbon by treating said gaseous reaction mixture with a solvent, which comprises bringing the gaseous reaction mixture which contains the maleic anhydride into contact with dibutyl succinate or fumarate in countercurrent, and removing the liquid product from the bottom product.

2. A process as claimed in claim 1, wherein from 0.2 to 10 moles of butanol and from 0.2 to 20 moles of dibutyl succinate or fumarate are employed per mole of maleic anhydride.

3. A process as claimed in claim 1, wherein the gaseous reaction mixture which contains the maleic anhydride and the butanol are introduced into the bottom part of a column, water is withdrawn from the column at a point above said point of introduction, the dibutyl succinate or fumarate is introduced at the top of the column, and the liquid product is removed from the base of the column.

4. A process as claimed in claim 1, wherein the butanol and the gaseous reaction mixture which contains the maleic anhydride are introduced into the bottom part of a column, a liquid mixture is withdrawn from the middle part of the column, lower boilers than dibutyl succinate or fumarate are distilled out of said mixture and introduced into the bottom part of the column, and the dibutyl ester obtained as a bottom product in this distillation is introduced at the top of the column.

**Revendications**

1. Procédé pour séparer en continu l'anhydride maléique de mélanges réactionnels gazeux que l'on obtient par oxydation catalytique d'hydrocarbures, par traitement de ces mélanges réactionnels gazeux par des solvants, caractérisé en ce qu'on met en contact avec du butanol le mélange réactionnel gazeux contenant l'anhydride maléique, on met en contact à contre-courant les substances gazeuses ainsi obtenues avec de l'ester dibutylique d'acide succinique ou de l'ester dibutylique d'acide fumarique et on prélève en bas de colonne le produit liquide du traitement.

2. Procédé selon la revendication 1, caractérisé en ce qu'on utilise, par rapport à 1 mole d'anhydride maléique, de 0,2 à 10 moles de butanol et de 0,2 à 20 moles d'ester dibutylique d'acide succinique ou fumarique.

3. Procédé selon la revendication 1, caractérisé en ce qu'on envoie dans la partie inférieure d'une colonne le butanol et le mélange réactionnel gazeux contenant l'anhydride maléique, on écluse de l'eau hors de la colonne au-dessus de cette admission, on introduit l'ester dibutylique d'acide succinique ou fumarique en tête de la colonne et on prélève en bas de colonne le produit liquide du traitement.

4. Procédé selon la revendication 1, caractérisé en ce qu'on envoie dans la partie inférieure d'une colonne le butanol et le mélange réactionnel contenant l'anhydride maléique, on écluse un mélange liquide hors de la partie moyenne de la colonne, on extrait par distillation de ce mélange et on envoie dans la partie inférieure de la colonne les fractions dont le point d'ébullition est plus bas que celui de l'ester dibutylique d'acide succinique ou fumarique et on introduit en tête de la colonne l'ester dibutylique obtenu en tant que résidu de cette distillation.